# EUROPEAN PATENT SPECIFICATION

(11) **EP 1 998 842 B1**
(45) Date of publication and mention of the grant of the patent: **24.10.2012**
(21) Application number: 07753574.8
(22) Date of filing: 20.03.2007
(51) Int. Cl.: A61M 37/00

(54) **VENOUS ACCESS PORT BASE**
VENENZUGANGSÖFFNUNGSBASIS
BASE POUR ORIFICE D'ACCÈS VEINEUX

(30) Priority: 29.03.2006 US 786976 P
(43) Date of publication of application: 10.12.2008
(73) Proprietor: Medical Components, Inc., Harleysville, PA 19438 (US)
(72) Inventor: BIZUP, Raymond, Feasterville, PA 19053 (US); SANFORD, Kevin, Chalfont, PA 18914 (US)
(74) Representative: Teasdale, Nicola Joanne
(86) International application number: PCT/US2007/006957
(87) International publication number: WO 2007/126645

(56) References cited:
- DE-A1- 4 225 524
- DE-U1- 29 512 576
- US-A- 3 211 431
- US-A- 5 951 512
- US-A- 6 113 572

## Description

### FIELD OF THE INVENTION

This relates to the field of medical devices and more particularly to venous access ports.

### BACKGROUND OF THE INVENTION

Venous access ports for the infusion and/or withdrawal of fluids from a patient are well-known. These devices are typically used for drug infusion or small amounts of blood aspiration. Where large flows of fluid are required, larger ports can be used such as for hemodialysis or plasmapheresis.

Some ports may be implanted subcutaneously. Implantable venous access ports have the advantage that they can remain within the patient for prolonged periods of time, permitting multiple use and decreasing the risk for associated infection. These ports typically provide a septum defining an access site for multiple needle sticks without the need to continuously search for new access sites, since the septum is comprised of material such as silicone elastomer that self-seals each time as a needle is withdrawn. These ports also each include a stem, or discharge port, that extends through a distal wall and that has a passageway therethrough; the stem is secured to the proximal end of a catheter so that the discharge port passageway is in fluid communication with the catheter lumen. One such catheter infusion port is disclosed in U.S. Patent No. 6,113,572.
In US 5,951,512 there is described an implantable infusion port which comprises a fluid reservoir with inlet means, the reservoir preferably having a toroid-like shape and outlet means directed along a tangent to the reservoir, assures more effective reservoir cleansing when the port is flushed with aqueous saline between uses.

Other types of ports are in use, known as dual ports or multi-ports. These provide two or more internal septa and chambers, all corresponding to different lumens of the attached catheter via respective separate discharge ports or alternatively, separate passageways in a single stem for communication with separate lumens of a dual or multi-lumen catheter, such as in U.S. Patent No. 5,360,407.

J Such venous access ports are commonly circular in shape, or at least the chamber, or each chamber, is commonly circular, and the chamber floor is planar. As a result of the circular shape of a chamber and a planar chamber floor, the liquid injected during infusion exhibits a vortex behavior, and it has become noticeable that some liquid remains in the chamber by virtue of being near the center of the circular chamber's planar floor while the flow vortex occurs about the periphery of the chamber floor.

It is desirable to minimize the amount of liquid remaining in the chamber of a venous access port during infusion.

### BRIEF SUMMARY OF THE INVENTION

The present invention is a venous access port (100, 100A) comprising:
a port body (102) having a discharge port (108) and a needle penetrable septum (104) affixed thereto, together defining a chamber (112, 112A) therebetween in fluid communication with a passageway (110) through the discharge port (108), the needle-penetrable septum (104) affixed to the port body (102) to fluidly seal the chamber (112, 112A),
characterised in that:
   the chamber (112, 112A) comprises a vertical chamber side wall (118, 118A) and a chamber floor (114, 114A) wherein the chamber floor comprises a peripheral region and a central region and wherein the central region of the chamber floor is elevated with respect to the peripheral region of the chamber floor and further wherein the vertical chamber side wall (118, 118A) is straight in a vertical direction and is perpendicular to the chamber floor (114, 114A) and wherein the peripheral region of the chamber floor is radiused adjacent the vertical chamber side wall (118, 118A).
Preferably the chamber floor is convex or dome-shaped in the central region but the chamber floor may also be conical with a low- height apex and flat sloping side surfaces, and the chamber floor is preferably radiused adjacent the chamber side wall tangentially.

### BRIEF DESCRIPTION OF THE DRAWINGS

The accompanying drawings, which are incorporated herein and constitute part of this specification, illustrate the presently preferred embodiments of the invention, and, together with the general description given above and the detailed description given below, serve to explain the features of the invention. In the drawings:

Fig. 1 is an exploded isometric view of a venous access port containing the present invention;

Fig. 2 is a cross-sectional view of the assembled port of Fig. 1 illustrating the present invention; and

Fig. 3 is a cross-sectional view of an alternate embodiment of the present invention.

### DETAILED DESCRIPTION OF THE INVENTION

In the drawings, like numerals indicate like elements throughout. Certain terminology is used herein for convenience only and is not to be taken as a limitation on the present invention. The terms "distal" and "proximal" refer, respectively, to directions closer to and away from the insertion tip of a catheter in an implantable catheter assembly. The terminology includes the words specifically mentioned, derivatives thereof and words of similar import. The embodiments illustrated below are not intended to be exhaustive or to limit the invention to the precise form disclosed. These embodiments are chosen and described to best explain the principle of the invention and its application and practical use and to enable others skilled in the art to best utilize the invention.

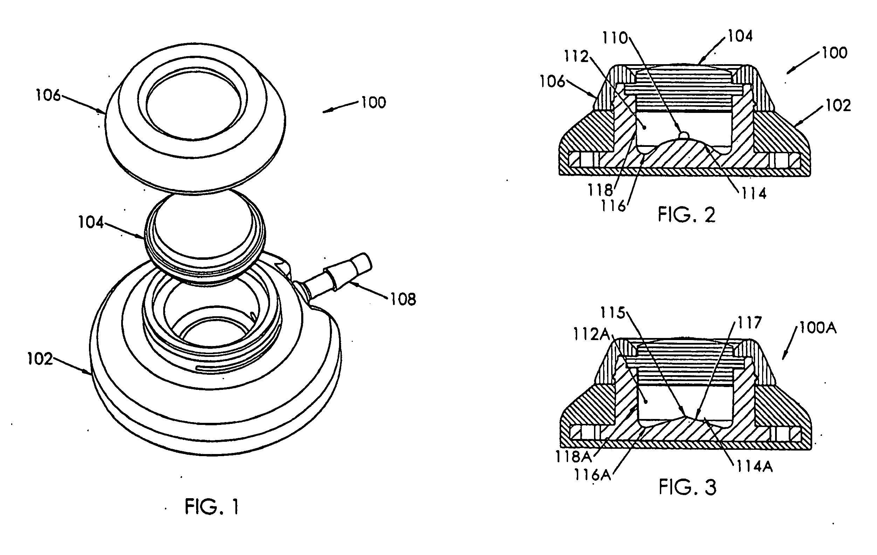

A first embodiment of venous access port 100 is illustrated in Figs. 1 and 2 and includes a base 102, a septum 104 and a retention cap 106 that secures the septum to the base. A discharge port 108 extends from the base to which the proximal end of a catheter (not shown) is to be affixed, and a passageway 110 extends through discharge port 108 into the catheter lumen from the chamber 112 that is defined between the septum 104 and the base 102. Septum 104 is made of a self-sealing material such as preferably silicone elastomer, such that an infusion needle can be inserted through the septum for infusion of medication and upon withdrawal of the needle from the septum, the septum will immediately seal the hole through which the needle had extended.

In Figure 2, chamber 112 is shown in cross-section. Chamber floor 114 is shown to be convex upwardly in the center of the chamber, and a vortex flow path 116 is seen to be defined about the periphery of the chamber. Passageway 110 is also seen that extends outwardly from the chamber and through the discharge port.

As a result of the convex shape of the chamber floor 114, infused medication will flow in its vortex flow path 116, and eventually as the level of fluid within the chamber diminishes, all liquid in the center of the chamber will perforce flow radially from the center toward the chamber side wall 118 and into the vortex path. With the elevated chamber floor of the present invention, clearance of the infused fluid is seen to begin sooner than with a flat floor design as no fluid can remain in the central region of the chamber floor.

Alternatively, as seen in Figure 3, the chamber floor 114A of a second embodiment of venous access port 100A may have a conical shape of low height, with an apical center point 115 and flat, sloping side surfaces 117 extending to the periphery of the chamber, again there defining a vortex flow path 116A, at which location the surfaces preferably are radiused to be tangent with the side wall 118A of the chamber.

It will be seen that it is preferred that the chamber floor of the present invention is elevated in its central region relative to the chamber floor periphery, and that the chamber floor has no level portions of substantial size. Variations of the present invention can easily be provided in chambers of dual chamber or multiple chamber venous access ports and achieve the benefits of the present invention of minimizing liquid remaining in the chamber after infusion.

## Claims

1. A venous access port (100, 100A) comprising:
a port body (102) having a discharge port (108) and a needle penetrable septum (104) affixed thereto, together defining a chamber (112, 112A) therebetween in fluid communication with a passageway (110) through the discharge port (108), the needle-penetrable septum (104) affixed to the port body (102) to fluidity seal the chamber (112, 112A),
**characterised in that**:
the chamber (112, 112A) comprises a vertical chamber side wall (118, 118A) and a chamber floor (114, 114A) wherein the chamber floor comprises a peripheral region and a central region and wherein the central region of the chamber floor is elevated with respect to the peripheral region of the chamber floor and further wherein the vertical chamber side wall (118, 118A) is straight in a vertical direction and is perpendicular to the chamber floor (114, 114A) and wherein the peripheral region of the chamber floor is radiused adjacent the vertical chamber side wall (118, 118A).

2. The venous access port (100, 100A) of claim 1 further **characterised in that** the chamber floor (114) is convex in the central region.

3. The venous access port (100, 100A) of claim 1 further **characterised in that** the chamber floor (114A) is conical upwardly to an apex (115) and has relatively flat sloping surfaces (117) extending to the peripheral region of the chamber floor.

4. The venous access port (100, 100A) of claim 3 further **characterised in that** the peripheral region of the chamber floor (114A) is radiused to join the chamber side wall (118A) tangentially.

5. The venous access port (100, 100A) of claim 1 further **characterised in that** the chamber floor (114,114A) has no level portion of substantial size.

## Patentansprüche

1. Venenzugangsanschluss (100, 100A), der Folgendes umfasst:
einen Anschlusskörper (102), der einen Ablassanschluss (108) und eine an demselben befestigte nadeldurchdringbare Scheidewand (104) hat, die zusammen eine Kammer (112, 112A) zwischen denselben in Fluidverbindung mit einem Durchgang (110) durch den Ablassanschluss (108) bilden, wobei die nadeldurchdringbare Scheidewand (104) an dem Anschlusskörper (102) befestigt ist, um die Kammer (112, 112A) fluiddicht abzudichten,
**dadurch gekennzeichnet, dass**:
die Kammer (112, 112A) eine vertikale Kammerseitenwand (118, 118A) und einen Kammerboden (114, 114A) umfasst, wobei der Kammerboden einen Umfangsbereich und einen Mittelbereich umfasst und wobei der Mittelbereich des Kammerbodens in Bezug auf den Umfangsbereich des Kammerbodens erhöht ist und wobei ferner die vertikale Kammerseitenwand (118, 118A) in einer vertikalen Richtung gerade ist und senkrecht zu dem Kammerboden (114, 114A) ist und wobei der Umfangsbereich des Kammerbodens angrenzend an die vertikale Kammerseitenwand (118, 118A) gerundet ist.

2. Venenzugangsanschluss (100, 100A) nach Anspruch 1, ferner **dadurch gekennzeichnet, dass** der Kammerboden (114) in dem Mittelbereich konvex ist.

3. Venenzugangsanschluss (100, 100A) nach Anspruch 1, ferner **dadurch gekennzeichnet, dass** der Kammerboden (114A) nach oben bis zu einem Scheitel (115) kegelförmig ist und verhältnismäßig flache abfallende Flächen (117) hat, die sich bis zu dem Umfangsbereich des Kammerbodens erstrecken.

4. Venenzugangsanschluss (100, 100A) nach Anspruch 3, ferner **dadurch gekennzeichnet, dass** der Umfangsbereich des Kammerbodens (114A) gerundet ist, um sich tangential mit der Kammerseitenwand (118A) zu verbinden.

5. Venenzugangsanschluss (100, 100A) nach Anspruch 1, ferner **dadurch gekennzeichnet, dass** der Kammerboden (114, 114A) keinen ebenen Abschnitt von wesentlicher Größe hat.

## Revendications

1. Orifice d'accès veineux (100, 100A), comprenant :
un corps d'orifice (102), comportant un orifice de décharge (108) et une membrane à pénétration par une aiguille (104) qui y est fixée, définissant ensemble une chambre (112, 112A) entre eux, en communication de fluide avec un passage (110) à travers l'orifice de décharge (108), la membrane à pénétration par une aiguille (104) étant fixée sur le corps de l'orifice pour assurer l'étanchéité au fluide de la chambre (112, 112A) ;
**caractérisé en ce que** :
la chambre (112, 112A) comprend une paroi latérale verticale de la chambre (118, 118A) et un plancher de la chambre (114, 114A), le plancher de la chambre comprenant une région périphérique et une région centrale, la région centrale du plancher de la chambre étant surélevée par rapport à la région périphérique du plancher de la chambre, la paroi latérale verticale de la chambre (118, 118A) étant en outre droite dans une direction verticale et perpendiculaire au plancher de la chambre (114, 114A), la région périphérique du plancher de la chambre étant arrondie près de la paroi latérale verticale de la chambre (118, 118A).

2. Orifice d'accès veineux (100, 100A) selon la revendication 1, **caractérisé en outre en ce que** le plancher de la chambre (114) est convexe dans la région centrale.

3. Orifice d'accès veineux (100, 110A) selon la revendication 1, **caractérisé en outre en ce que** le plancher de la chambre (114A) est conique vers le haut, en direction d'un sommet (115), et comporte des surfaces inclinées relativement plates (117) s'étendant vers la région périphérique du plancher de la chambre.

4. Orifice d'accès veineux (100, 100A) selon la revendication 3, **caractérisé en outre en ce que** la région périphérique du plancher de la chambre (114A) est arrondie en vue d'une liaison tangentielle à la paroi latérale de la chambre (118A).

5. Orifice d'accès veineux (100, 100A) selon la revendication 1, **caractérisé en outre en ce que** le plancher de la chambre (114, 114A) ne comporte pas de partie nivelée de taille substantielle.
